Europäisches Patentamt

European Patent Office

Office européen des brevets

(19)

(11) Publication number: **0 121 966**
**A1**

(12) **EUROPEAN PATENT APPLICATION**

(21) Application number: **84200334.5**

(22) Date of filing: **09.03.84**

(51) Int. Cl.³: **A 61 F 13/18, A 41 B 13/02**

(30) Priority: **17.03.83 BE 210338**

(43) Date of publication of application: **17.10.84**
**Bulletin 84/42**

(84) Designated Contracting States: **AT CH DE FR GB IT LI LU NL SE**

(71) Applicant: **Schoofs, Frans, Zeedijk Albertstrand, 568-bus 17, B-8300 Knokke-Heist (BE)**

(72) Inventor: **Schoofs, Frans, Zeedijk Albertstrand, 568-bus 17, B-8300 Knokke-Heist (BE)**

(74) Representative: **Pieraerts, Jacques et al, Bureau Gevers S.A. rue de Livourne 7, Bte. 1, B-1050 Bruxelles (BE)**

(54) **Sanitary towel, throw-away napkin or similar object, with improved sanitary properties.**

(57) The invention relates to a sanitary towel, throw-away baby napkin or similar object, which is comprised of a strip absorbing material (1) with one-sided self-adhesive edges (3,3' and 4,4') projecting at least sidewise along the lengthwise direction of said strip, in such a way that after being used, the towel may be folded in the middle and along the cross-wise direction, while the self-adhesive edges (3–3' or 4–4') after folding over 180° outwards are pressed against one another.

EP 0 121 966 A1

"Sanitary towel, throw-away napkin or similar object, with improved sanitary properties".

This invention relates to a sanitary towel, a throw-away napkin, or a similar object with improved sanitary properties.

The object of the invention is to provide a sanitary towel which is so structured that after being used and in folded condition and as it were packed, it may be thrown-away.

For this purpose, the sanitary towel according to the invention is comprised of a strip absorbing material with edges projecting at least sidewise along the lengthwise direction of said strip, which are self-adhesive on the one side, all this in such a way that after being used, the towel may be folded in the middle and along the cross-wise direction, while the self-adhesive edges after being folded over 180° out-wards, may be pressed together whereby the towel may be thrown-away in folded condition.

According to an advantageous embodiment of the invention, said self-adhesive projecting edges are part of a sheet impermeable material which is joined to said absorbing material strip.

Advantageously, projecting one-sided self-adhesive edges are also provided along both cross-wise ends of said absorbing material strip.

Other details and advantages of the invention will stand out from the following description, given by way of non-limitative example and with reference to the accompanying drawings, in which :

Figure 1 is a perspective showing of a sanitary towel according to the invention, as seen from the back.

Figure 2 is a perspective showing of a sanitary towel as shown in figure 1, in the condition wherein it may be folded after being used.

Figure 3 is a showing of the sanitary towel in a further folding position.

The sanitary towel according to these figures is comprised in an embodiment which is to be preferred, but which is only described by way of example, of a strip 1 from absorbing material and a sheet 2 connected thereto from impermeable material. The connection between the strip 1 and the sheet 2 is obtained by using some method which makes it possible to bind cellulose-like materials to a sheet 2 for example from polymer material.

One such method lies simply in glueing the strip 1 to the sheet 2.

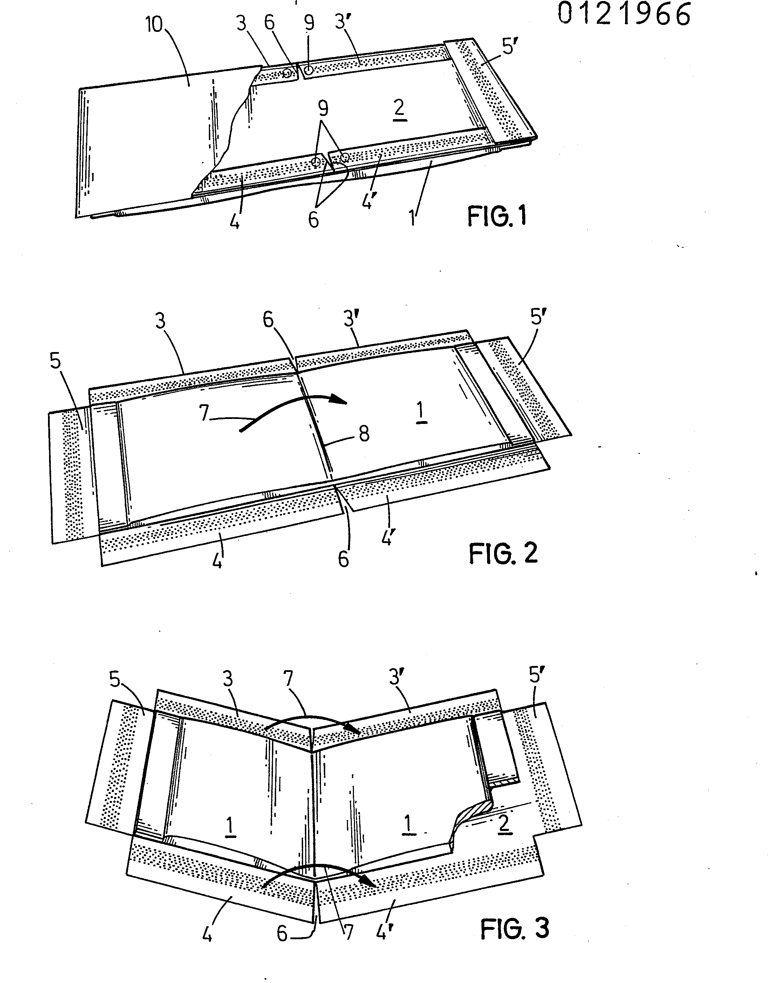

This sheet 2 has as well along the lengthwise direction as along the cross-wise direction, self-adhesive edges which after being folded over 180° outwards, may be considered as projecting edges. To make the description clear, the self-adhesive edges which appear as projecting edges on either side of the strip 1 are indicated with the references 3-3' and 4-4'. The self-adhesive edges which lie at both ends of the strip 1, that is the self-adhesive projecting edges after folding over 180° outwards and which lie in the cross-wise direction, are indicated with the references 5-5'. These edges are provided over the whole surface thereof or locally with an adhesive material. Between the edges 3-3' and 4-4' may occur a cut-in to enhance folding. Adhesive spots 9 connect temporarily and locally said edges with the sheet 2 said edges are folded back on. Figure 1 which shows the towel as it is supplied and before being used, shows this clearly.

The edges 5-5' which are folded inwards, retain the edges 3-3' and 4-4' in the inward-folded condition as shown in figure 1, suitably in position.

Over the sheet 2 as well as over the folded edges 3-3', 4-4' and 5-5' is provided a silicone paper sheet 10 which is first removed when using the sanitary towel. In figure 1, said silicone paper sheet has been partly drawn, so that this figure pertains both to the sanitary towel as it is supplied, that is with the silicone paper sheet, as to the condition wherein it is being used after removing said silicone paper sheet. Figure 1 thus shows the back side of the sanitary towel as opposed to figures 2 and 3 which show the sanitary towel as reversed relative to figure 1, with the absorbing material strip visible on the top side.

In the condition of use, the self-adhesive edges 3-3', 4-4' and 5-5' thus lie on the other side relative to the absorbing material strip 1.

The self-adhesive edges thus adhere well to fabrics and retain the towel in position, as it is used.

After being used, the self-adhesive edges are very easily brought to the position wherein they are shown in figures 2 and 3. The self-adhesive edges 3 and 4 are easily released from the sheet 2 in the location of the adhesive spots 9 to then become projecting edges. When folding-over the cross-wise edges 5-5', the edges 3-3' and 4-4" are also released so that these self-adhesive edges then take the position or shown in figures 2 and 3.

The towel is then folded in the direction as shown by arrows 7 along the fold line 8, level with the two incisions 6.

When the sanitary towel is completely folded, that is when the self-adhesive projecting edges 3 and 3' on the one side, and 4 and 4' on the other side, as well as the projecting cross edges 5 and 5' are in contact with one another, the sanitary towel lies as completely "wrapped". The absorbing strip 1 lies then completely enclosed in the impermeable sheet 2, so that the throwing-away of the towel does not raise any problem.

Even when the above description refers completely to a sanitary towel, the invention is naturally also to be applied for throw-away baby napkins and similar objects.

It is further clear that the invention is not limited to the above-described embodiment and that many changes may be brought therein, notably as regards the shape, composition and arrangement of the elements which are essential to embodying the invention.

## CLAIMS.

1. Sanitary towel, throw-away baby napkin or similar object, which is comprised of a strip absorbing material (1) with one-sided self-adhesive edges (3,3' and 4,4') projecting at least sidewise along the lengthwise direction of said strip, in such a way that after being used, the towel may be folded in the middle and along the cross-wise direction, while the self-adhesive edges (3-3' or 4-4') after folding over 180° outwards are pressed against one another, whereby the towel may be thrown-away in closed condition.

2. Sanitary towel as defined in claim 1, in which said projecting self-adhesive edges (3-3' and 4-4') are part of a sheet impermeable material (2) which is joined to said absorbing material strip (1).

3. Sanitary towel as defined in either one of claims 1 and 2, in which cross-wise edges (5-5') are also provided, such edges being self-adhesive on the one side thereof and comprising after folding over 180° outwards, projecting edges.

4. Sanitary towel as defined in any one of claims 1-3, in which the self-adhesive edges (3-3' and 4-4') are folded over on said sheet (2) and retained contacting same by glue spots (9).

5. Sanitary towel as defined in any one of claims 1-4, in which said cross-wise edges (5-5') are folded over the ends of the self-adhesive edges (3-3' and 4-4').

6. Sanitary towel as defined in any one of claims 1-5, in which the self-adhesive edges (3-3') on the one side and (4-4') on the other side are separated from one another level with a fold line (8) by means of an incision (6).

7. Sanitary towel as defined in any one of claims 1-6, in which the self-adhesive edges (3-3', 4-4' and 5-5') are covered with a silicone paper sheet (10).

FIG. 1

FIG. 2

FIG. 3

**European Patent Office**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | FR-A-2 490 952 (P. HARTMANN AG) * Page 4, lines 7-15; page 6, lines 1-17, figures * | 1,2 | A 61 F 13/18 A 41 B 13/02 |
| Y | | 3 | |
| Y | FR-A-2 086 742 (R. SCHWOB et al.) * Page 2, lines 24-30; page 4, lines 26-28; figures 1,4 * | 3 | |
| A | DE-A-2 707 350 (VEREINIGTE PAPIERWERKE SCHICKEDANZ & CO.) * Pages 7,8; figures 1,2 * | 2,4,7 | |

TECHNICAL FIELDS SEARCHED (Int. Cl. ³)

A 61 F
A 41 B

The present search report has been drawn up for all claims

| Place of search THE HAGUE | Date of completion of the search 20-06-1984 | Examiner GRENTZIUS W. |
|---|---|---|